# EUROPEAN PATENT SPECIFICATION

(11) **EP 4 167 768 B1**
(45) Date of publication and mention of the grant of the patent: **09.07.2025**
(21) Application number: 21732918.4
(22) Date of filing: 18.06.2021
(51) Int. Cl.: A23L 33/10, A23L 33/00

(54) **A NUTRITIONAL COMPOSITION COMPRISING 3-HYDROXYBUTYRIC ACID TO IMPROVE THE GASTROINTESTINAL BARRIER**
ERNÄHRUNGSZUSAMMENSETZUNG MIT 3-HYDROXYBUTTERSÄURE ZUR VERBESSERUNG DER MAGEN-DARM-BARRIERE
COMPOSITION NUTRITIONNELLE COMPRENANT DE L'ACIDE 3-HYDROXYBUTYRIQUE POUR AMÉLIORER LA BARRIÈRE GASTRO-INTESTINALE

(30) Priority: 19.06.2020 EP 20181187
(43) Date of publication of application: 26.04.2023
(73) Proprietor: Société des Produits Nestlé S.A., 1800 Vevey (CH)
(72) Inventor: GARCIA-RODENAS, Clara, Lucia, 1072 Forel (CH); NATÎVIDAD, Jane, Mea, M., 1018 LAUSANNE (CH); RYTZ, Andreas, 1084 CARROUGE (CH)
(74) Representative: Elkington and Fife LLP
(86) International application number: PCT/EP2021/066562
(87) International publication number: WO 2021/255229

(56) References cited:
- WO-A1-2020/013683
- WO-A1-2020/070087
- WO-A1-2020/126587
- US-A1- 2010 093 860
- US-A1- 2016 095 339
- US-A1- 2019 262 415
- US-A1- 2019 380 982
- QINGDING WANG ET AL: "Ketogenesis contributes to intestinal cell differentiation", CELL DEATH & DIFFERENTIATION, vol. 24, no. 3, 9 December 2016 (2016-12-09), GB, pages 458 - 468, XP055751377, ISSN: 1350-9047, DOI: 10.1038/cdd.2016.142
- J.E. AGUILAR-TOALÁ ET AL: "Postbiotics: An evolving term within the functional foods field", TRENDS IN FOOD SCIENCE AND TECHNOLOGY, vol. 75, 1 May 2018 (2018-05-01), GB, pages 105 - 114, XP055592436, ISSN: 0924-2244, DOI: 10.1016/j.tifs.2018.03.009
- PATEL RAVI MANGAL ET AL: "Therapeutic Use of Prebiotics, Probiotics, and Postbiotics to Prevent Necrotizing Enterocolitis What is the Current Evidence?", CLINICS IN PERINATOLOGY, W.B. SAUNDERS CO, US, vol. 40, no. 1, 1 March 2013 (2013-03-01), pages 11 - 25, vii, XP009185531, ISSN: 0095-5108, DOI: 10.1016/J.CLP.2012.12.002
- VAN T. PHAM ET AL: "The effects of fermentation products of prebiotic fibres on gut barrier and immune functions in vitro", PEERJ, vol. 6, 10 August 2018 (2018-08-10), pages e5288, XP055592800, DOI: 10.7717/peerj.5288
- SANTSCHI D E ET AL: "Prevalence of elevated milk [beta]-hydroxybutyrate concentrations in Holstein cows measured by Fourier-transform infrared analysis in Dairy Herd Improvement milk samples and association with milk yield and components", JOURNAL OF DAIRY SCIENCE, vol. 99, no. 11, 1 March 2016 (2016-03-01), pages 9263 - 9270, XP029776230, ISSN: 0022-0302, DOI: 10.3168/JDS.2016-11128

## Description

### Technical field of the invention

The present invention relates to nutritional compositions comprising 3-hydroxybutyric acid for use in improving gastrointestinal barrier. In particular, the present invention relates to improving the gastrointestinal barrier in an individual, preferably in an infant or a child.

### Background of the invention

The gastrointestinal barrier plays an important role as a functional barrier to macromolecules and pathogenic organisms, while allowing for the absorption of nutrients, such as minerals and vitamins. The intestinal epithelial barrier is the first line that separates the inside of the body from the outside environment, including the microbiota, dietary components and environmental toxins. A proper functioning gut epithelial barrier is crucial for the maintenance of overall wellbeing and in regulating a diverse range of functions including immunity and hormonal balance and in return can affect a wide variety of health and clinical outcomes. Considering the far-reaching impact of the gut epithelial barrier on overall health, there is a need for compositions able to provide health benefits such as improving, enhancing or protecting the gut barrier function, such as to optimize health and general wellbeing of an individual.

The need is particularly acute in infants and young children. During the postnatal development, the new-born intestine experiences a process of maturation that ends by the establishment of a functional barrier. This phenomenon is called gut closure and appears to be affected by the diet. Hence, different studies with infants (JPGN, 1995, 21: 383-6) and animal models (Pediatr Res, 1990, 28: 31-7) show that the maturation of the barrier is faster in breast-fed than in formula-fed new-borns. This could explain the higher prevalence of allergy and infection in infants fed formula than in those fed with mother milk.

Mother's milk is recommended for all infants. However, in some cases breast feeding is inadequate or unsuccessful for medical reasons or the mother chooses not to breast feed. Infant formulae have been developed for these situations. Fortifiers have also been developed to enrich mother's milk or infant formula with specific ingredients. In such cases, it would be even more preferred to provide means to improve gastrointestinal barrier in infants and young children through nutritional intervention, such as through complete nutrition or nutritional supplements.

Some specific populations of infants and young children are particularly in need of compositions able to provide health benefits such as improving gut barrier function. Such infants and young children are for example preterm infants, low birth weight infant, and/or growth-retarded infants or young children. Indeed the gut barrier is more permeable and more susceptible to injury and its structure and function are less mature in such infants than in a healthy term infant. This in turn may lead to other problems such as infection or allergy. For such infants it is particularly advantageous to complement pharmacological management with nutritional compositions capable of improving gut barrier function. New-born and young animals, such as pet animals may also be at need for improvement of the gut barrier for the same reasons.

The intestinal barrier can be also compromised in older children and adults such as those suffering from gastro-intestinal disease (e.g. Inflammatory bowel disease, chronic diarrhea) and/or critically ill and/or receiving parenteral nutrition. The same applies to animals, such as pet animals suffering from these conditions.

Hence, an improved and in particular a more efficient and/or reliable nutritional composition for improving gut barrier would be advantageous.

The effect of nutritional ingredients to improve gastrointestinal barrier has previously been investigated. This is namely the case of polyamines, which have been the object of a number of studies. For example A.F. Bekebrede, J. keijer, W.J.J. Gerrits and V.C.J. de Boer; The Molecular and Physiological Effects of Protein-Derived Polyamines in the Intestine; Nutrients, 2020, 12, pp 197ff provides a review of the effect of polyamines (namely spermine, spermidine, putrescine and cadaverine) on biochemical, cellular and physiological processes with a focus on the colon. Polyamines are described as supporting gut physiology, by supporting barrier function, inducing gut maturation and increasing longevity.

3-Hydroxybutyric acid has been previously described as having a potential beneficial effect on intestinal cell maturation (Q. Wang, Y. Zhou, P. Rychahou, T.W-M. Fan, A.N. Lane, H.L.Weiss and B.M. Evers, Ketogenesis contributes to intestinal cell differentiation, Cell Death and Differentiation (2017) 24, 458-468). However this document is silent with respect to intestinal barrier function, structure, protection and repair.

Other ingredients such as probiotics and prebiotics, such as human milk oligosaccharides (HMOs) have been identified as having positive effects on gut barrier.

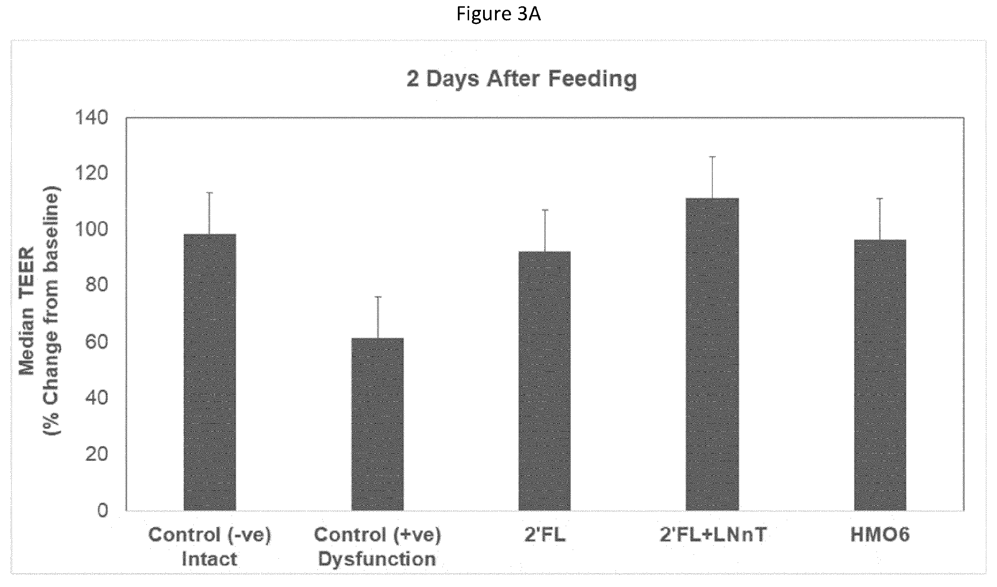

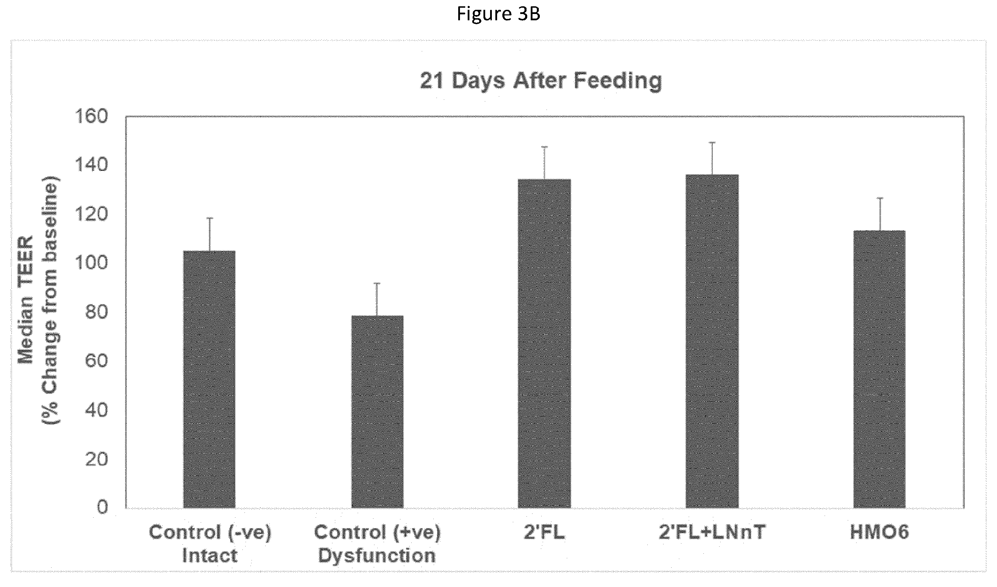

Human milk oligosaccharides are non-digestible oligosaccharides and thus cannot be metabolized by the enzymes produced by an infant or a young child. These oligosaccharides are however faced with the bacteria of the microbiota in the infant or young child gastrointestinal tract and are metabolized by such bacteria. The metabolism of HMOs in the infant gastrointestinal tract has been previously investigated. Bacteria metabolize HMOs by two different classes of mechanisms, depending on the genus/species of bacteria. *Bifidobacterium longum* subsp *infantis* internalizes the HMOs in their native form, without digesting them to smaller fragments and then metabolizes the HMO by an intracellular mechanism. By this mechanism, *B. infantis* releases important metabolites such as acetic acid and lactic acid into the gastrointestinal tract of the infant. Such metabolites are beneficial to the growth of other bacteria in the microbiota, such as *Bifidobacterium bifidum. Bifidobacterium bifidum* has a very different way of metabolizing HMOs. This bacterial species indeed releases enzymes that digest the HMOs through an extra-cellular mechanism and thus cleaves the HMOs into smaller fragments in the gastrointestinal tract of the baby, such smaller fragments being then available for consumption by the whole diversity of bacterial species in the microbiota. For example Sela et al.; Nursing our microbiota: molecular linkages between bifidoacteria and milk oligosaccharides; Trends Microbiol, 2010, 18(7): 298-307 describes in details the diverse mechanisms used by bacteria of the microbiota to metabolize HMOs. In particular, Figure 3 provides a graphic representation of such mechanisms.

It would be particularly advantageous to provide improved nutritional compositions, and in particular a more efficient and/or reliable nutritional composition for infants and young children having an impaired microbiota, such as for example formula-fed infant and even more preterm infants, infants born by caesarean section and infants and young children who have had or are having an antibiotic treatment. For example Chemikova et al.; The premature infant gut microbiome during the first 6 weeks of life differs based on gastrointestinal maturity at birth, Pediatric Research, 2018, 84: 71-79 shows how different the microbiota of a preterm infant is compared to a term infant (see for example Table 1 on page 72). Also Korpela et al.; Early life colonization of the human gut: microbes matter everywhere; Current Opinion on Microbiology,2018, 44: 70-78 provides a meta-analysis of the many studies assessing infant microbiota and extracts ranges for the amount of the five major genera present in infant microbiota for ages between birth and two years, for term vaginally-born infants (breast-fed and formula-fed), infants born by caesarean section and infants treated with antibiotics. This publication reveals that the microbiota of caesarean section infants and infants treated with antibiotics have a microbiota that significantly differs from term infants who were born vaginally.

Several publications also highlight a significant difference between the microbiota of infants fed infant formula compared to breast-fed infants. See for example Lee et al.; Comparison of the gut microbiota profile in breast-fed and formula-fed Korean infants using pyrosequencing; Nutrition Research and Practice, 2015, 9(3):242-248.

WO2020126587 relates to nutritional compositions comprising metabolites of HMOs to improve the gastrointestinal barrier, particularly, for infants with impaired microbiota since their microbiota may not be able to metabolize HMOs.

Van T. Pham et al.; The effects of fermentation products of prebiotic fibres on gut barrier and immune functions in vitro; PeerJ, 2018, 6(e5288), tests the effects of five common dietary fibres after fermentation by human gut microbiota in vitro, on measures of gut barrier integrity.

The typical microbiota of breast-fed infants is known to be particularly efficient in metabolizing HMOs. It would be useful to further improve the effect of nutritional compositions on the health of all infants and children and in particular in infants and young children having an impaired microbiota, which cannot metabolize HMOs in an optimal way. There is clearly a need for developing suitable methods to improve gastrointestinal barrier in infants and young children and in particular in infants and young children having an impaired microbiota.

It would be useful to further optimize the effect of nutritional compositions on gastrointestinal barrier in all individuals, and in particular in all infants and children. There is clearly a need for developing suitable methods to improve gastrointestinal barrier in an individual and in particular in infants and young children.

There is also a need to deliver such health benefits in a manner that is particularly suitable for the young or fragile subjects (such as subjects with impaired health, infants and young children), that does not involve a classical pharmaceutical intervention.

There is a need to deliver such health benefits in these subjects in a manner that does not induce side effects and/or in a manner that is easy to deliver, and well accepted by the parents or health care practitioners.

There is also a need to deliver such benefits in a manner that does keep the cost of such delivery reasonable and affordable by most.

Thus, there is clearly a need to develop alternative methods than the classical pharmaceutical intervention such as the use of pharmaceuticals, at least because of the associated risk of side effects.

### Summary of the invention

The present invention relates to nutritional compositions comprising 3-hydroxybutyric acid for use in improving the gastrointestinal barrier in a subject; wherein said use is selected from the groups consisting of improving the prevention of barrier dysfunction, the prevention of barrier leakiness, the protection of tight junction structure and the protection of the intestinal epithelial lining integrity; and/or wherein the subject has an impaired microbiota; wherein 3-hydroxybutyric acid is present in an amount of 0.01 mg/L to 10 g/L of the composition, or wherein 3-hydroxybutyric acid is present in an amount of 0.007 mg/100 g to 7 g/100 g of the composition on a dry weight basis; or wherein 3-hydroxybutyric acid is present in an amount of 0.003 mg to 3 g per serving. The effect is evidenced by the results of the in-vitro study on intestinal epithelium cell model.

3-hydroxybutyric acid is a degradation product of HMOs, namely of 2-fucosyllactose (2'FL). It is advantageous to use 3-hydroxybutyric acid as an active ingredient in place of HMOs from which it is derived. 3-Hydroxybutyric acid is formed in the gastrointestinal tract by the metabolism of HMOs and in particular 2-fucosyllactose (2'FL) by the gut flora. The rate of formation of 3-hydroxybutyric acid from HMOs by this biochemical process is however impacted by many parameters and it is an advantage to more precisely dose the amount of 3-hydroxybutyric acid that is provided in the gastrointestinal tract of the subjects, by adding this compound directly to the nutritional composition. In particular the metabolism of HMOs in the gastrointestinal tract is very much dependent on the gut flora that is present in the subject and the effect of HMOs that are mediated HMO metabolites is thus very much dependent on the composition of such flora as explained above. Therefore, direct administration of metabolites that are effective in improving the gut barrier are advantageous in that the effect can be obtained even in subjects having abnormal or otherwise impaired gut flora.

The present inventors have found improved nutritional compositions that are advantageous in that the individual consuming the composition does not need to possess microbiota capable of metabolizing the HMOs to be able to benefit from the effects of the HMOs on gut barrier. In other terms, also individuals having an impaired microbiota can get the full benefit.

Such compositions are particularly adapted to subjects having impaired microbiota, in particular infants and children, preferably infants who do not have a baby microbiota resembling the microbiota of a breast-fed infant, preferably of a vaginally born, term infant, and thus are at risk of metabolizing HMOs in a way that is not optimal. Such infants and young children are for example formula-fed infants, infants born by caesarean section, preterm infants and infants treated with antibiotics.

Thus, an object of the present invention relates to nutritional compositions, which improve the gastrointestinal barrier, such as improve barrier protection, improve barrier structure, improve barrier function, and/or barrier repair. Such aspects of gastrointestinal barrier are all interrelated. Proper gastrointestinal structure and function is maintained by proper barrier protection and restored by proper barrier repair. Thus, any of these aspects, considered together or individually, contribute to sustained reduction of disease susceptibility.

Thus, one aspect of the invention relates to a nutritional composition comprising 3-hydroxybutyric acid for use in improving the gastrointestinal barrier in a subject, preferably in an infant (i.e. a child under the age of 12 month) or in a child, such as a child between 1 and 8 years of age, wherein said use is selected from the groups consisting of improving the prevention of barrier dysfunction, the prevention of barrier leakiness, the protection of tight junction structure and the protection of the intestinal epithelial lining integrity; and/or wherein the subject has an impaired microbiota; wherein 3-hydroxybutyric acid is present in an amount of 0.01 mg/L to 10 g/L of the composition, or wherein 3-hydroxybutyric acid is present in an amount of 0.007 mg/100 g to 7 g/100 g of the composition on a dry weight basis; or wherein 3-hydroxybutyric acid is present in an amount of 0.003 mg to 3 g per serving.

In a preferred embodiment of the invention said improvement to the gastrointestinal barrier is improved barrier protection, barrier structure and barrier function, preferably barrier protection.

### Brief description of the figures

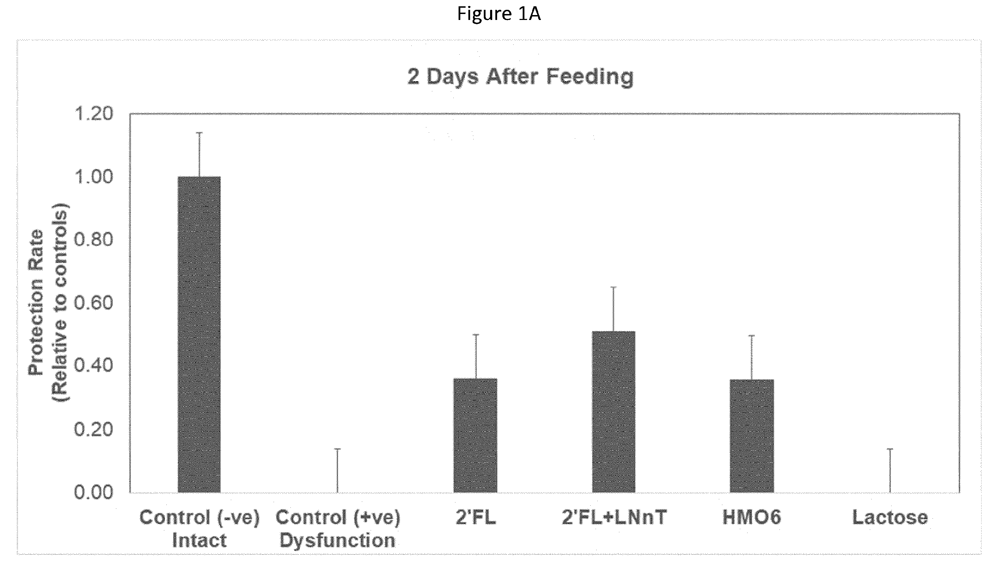

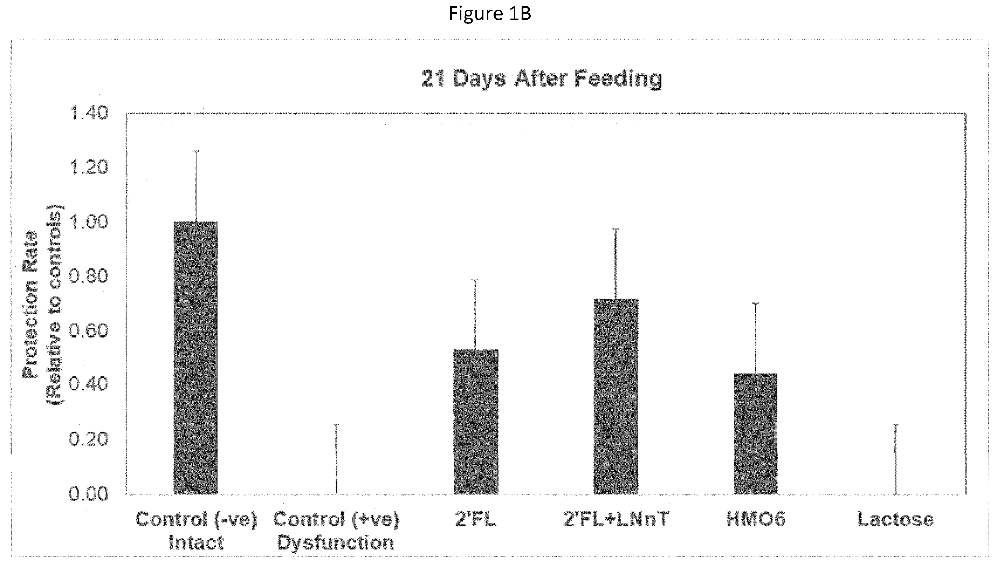

**Figure 1****: Efficacy of HMOs fermentation products to provide prophylactic epithelial barrier protection.** Co-cultures are treated with HMOs and then epithelial barrier dysfunction is induced by cytokine-mediated inflammation. All groups, in exception of control -ve (intact barrier), are inflammatory challenged. Protection rate is calculated by analyzing the evolution of transepithelial electrical resistance (TEER) relative to control -ve (100% protection) and control +ve (0% protection) before inflammatory challenge. Error bars represent LSD5%/2. **A)** 2 days of feeding. **B)** 21 days of feeding.

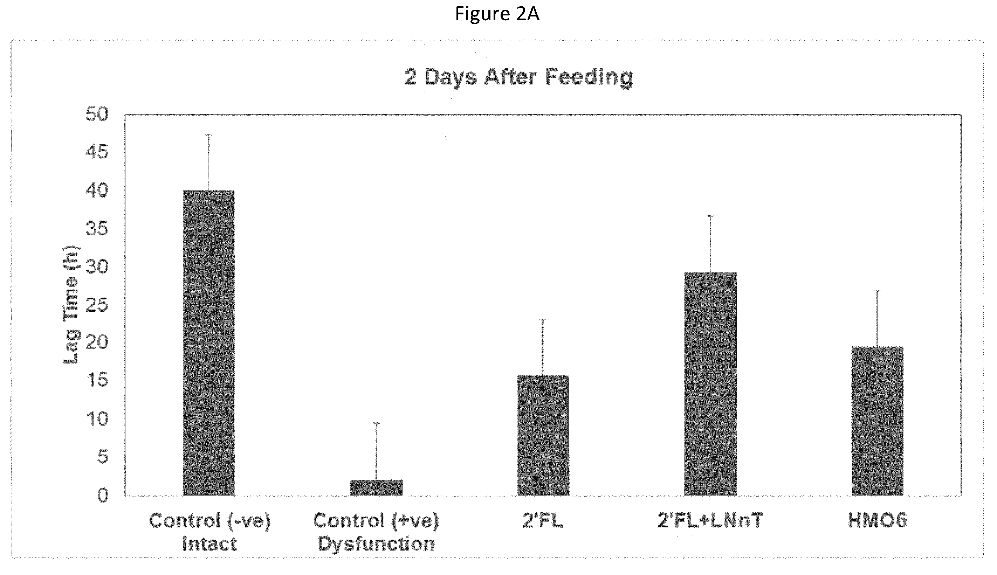

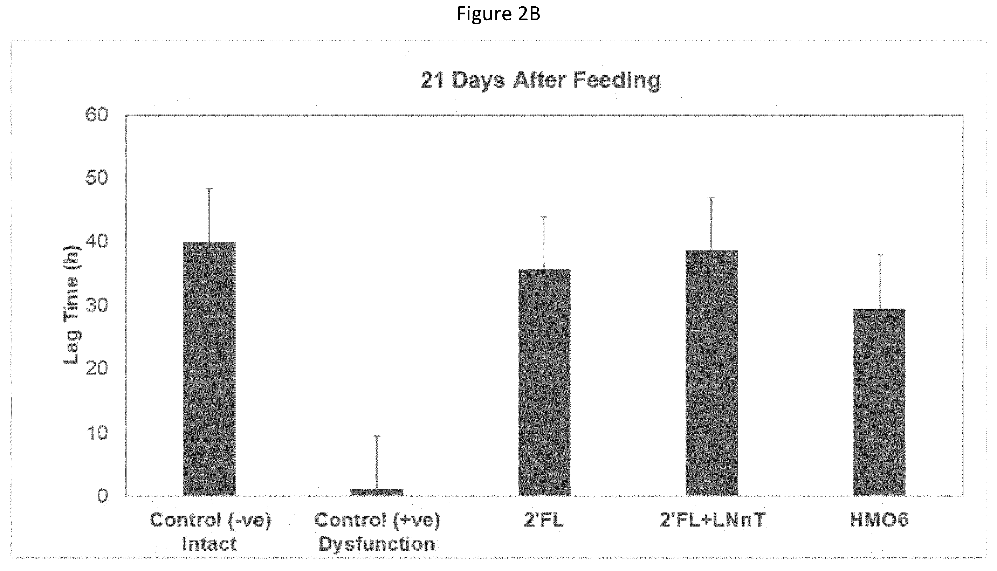

**Figure 2****: Efficacy of HMOs fermentation products to induce resistance against inflammation-induced epithelial barrier dysfunction.** Co-cultures are treated with HMOs and then epithelial barrier dysfunction is induced by cytokine-mediated inflammation. All groups, in exception of control -ve (intact barrier), are inflammatory challenged. Lag time is calculated as time it takes for transepithelial electrical resistance (TEER) to fall below control -ve (intact barrier) after the induction of inflammation. Error bars represent LSD5%/2. **A)** 2 days of feeding. **B)** 21 days of feeding.
**Figure 3****. Efficacy of HMOs fermentation products to limit susceptibility to inflammation-induced epithelial barrier dysfunction.** Co-cultures are treated with HMOs and then epithelial barrier dysfunction is induced by cytokine-mediated inflammation. All groups, in exception of control -ve (intact barrier), are inflammatory challenged. Graph represents median transepithelial electrical resistance (TEER) during inflammatory challenge. Error bars represent LSD5%/2. **A)** 2 days of feeding. **B)** 21 days of feeding.

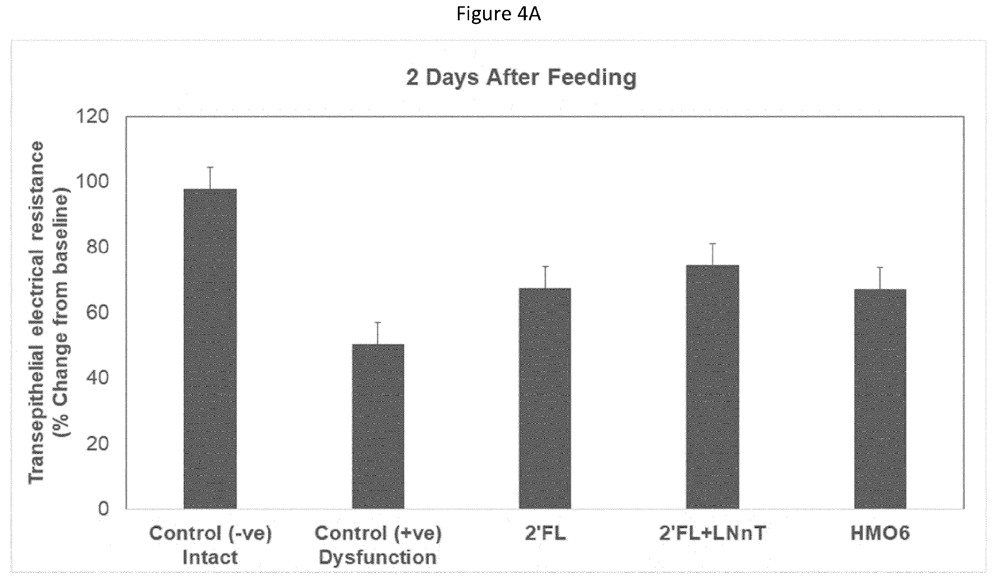

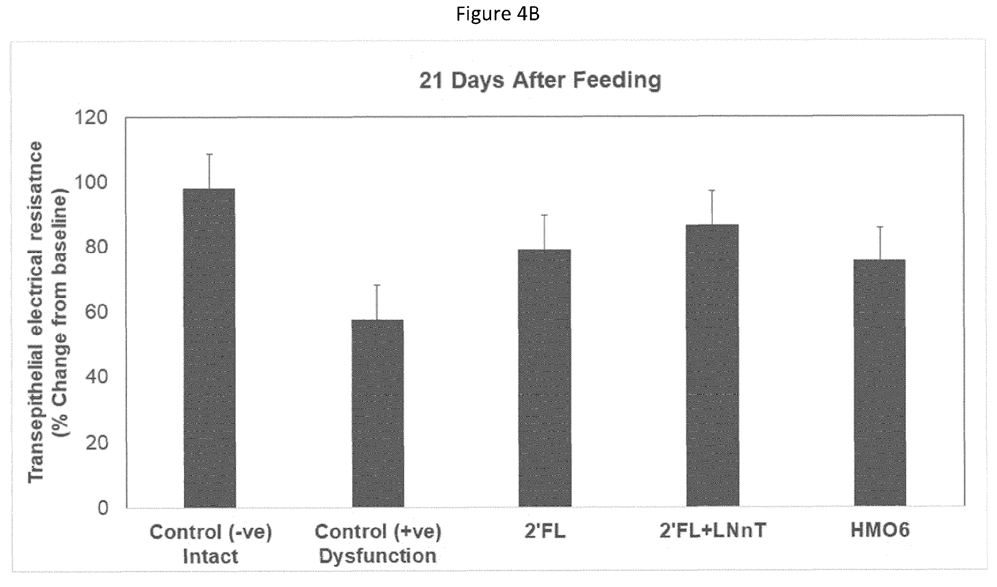

**Figure 4****: Efficacy of HMOs fermentation products to reduce symptoms severity of inflammation-induced epithelial barrier dysfunction.** Co-cultures are treated with HMOs and then epithelial barrier dysfunction is induced by cytokine-mediated inflammation. All groups, in exception of control -ve (intact barrier), are inflammatory challenged. The graph represents the final transepithelial electrical resistance (TEER) at the end of the inflammatory challenge. Error bars represent LSD5%/2. **A)** 2 days of feeding. **B)** 21 days of feeding.

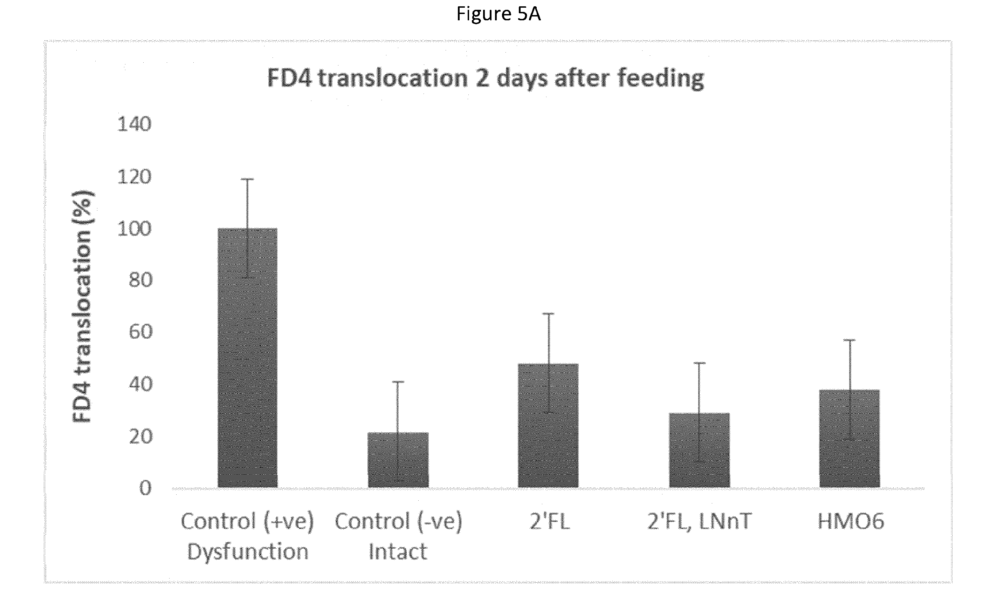

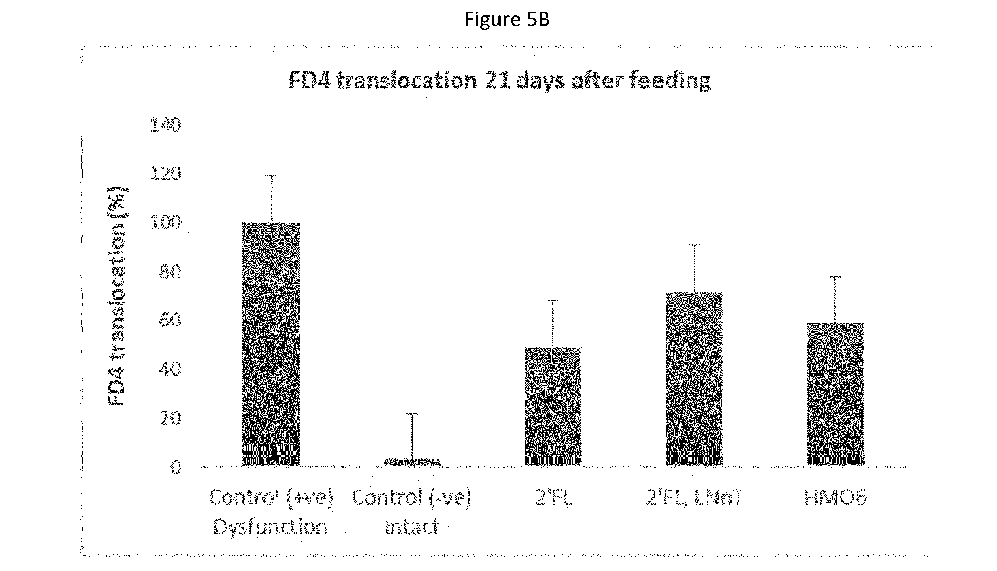

**Figure 5****: Efficacy of HMOs fermentation products to reduce symptoms severity of inflammation-induced epithelial barrier dysfunction.** Co-cultures are treated with HMOs and then epithelial barrier dysfunction is induced by cytokine-mediated inflammation. All groups, in exception of control -ve (intact barrier), are inflammatory challenged.
   Epithelial barrier intactness is calculated by analyzing the translocation of FITC-labeled dextran (FD4) from apical to basolateral compartment relative to control +ve (value set to 100; representing dysfunctional barrier) after inflammatory challenge.. Bars represent Mean values whereas intervals represent Mean+/- LSD5%/2 **A)** 2 days of feeding. **B)** 21 days of feeding.

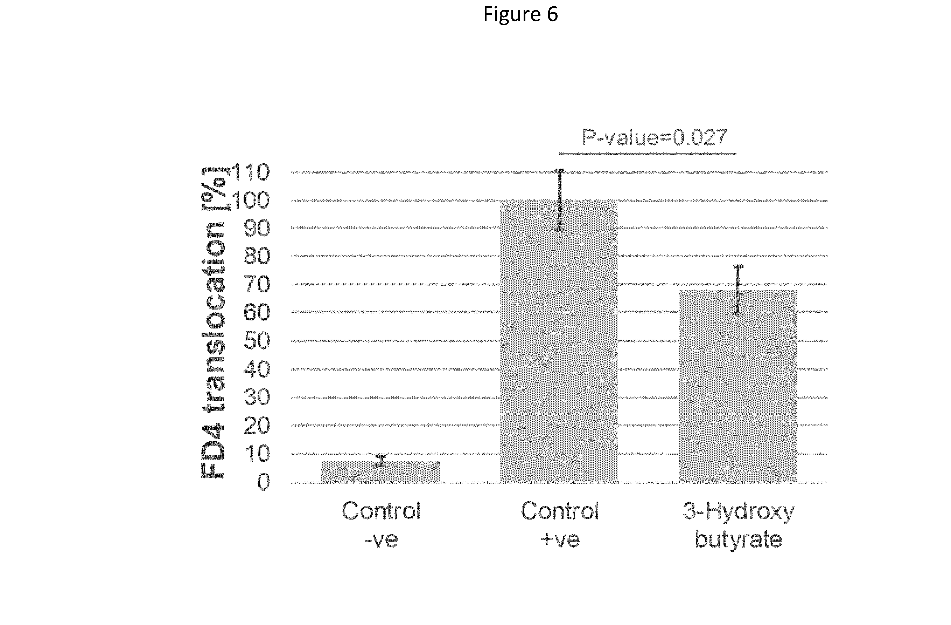

**Figure 6****: Efficacy of 3-hydroxybutyric acid to reduce inflammation-induced epithelial barrier dysfunction.** Co-cultures are treated with 3-hydroxybutyric acid and then epithelial barrier dysfunction is induced by cytokine-mediated inflammation. All groups in exception of control -ve (intact barrier) are inflammatory challenged. Epithelial barrier intactness is calculated by analyzing the translocation of FITC-labeled dextran (FD4) from apical to basolateral compartment relative to control control +ve (value set to 100; representing disfunctional barrier) after inflammatory challenge. Bars represent Mean values whereas intervals represent Mean+/- Standard Errors (SE). P-values were obtained through two-sided two-sample Student t-test assuming unequal variances.

The present invention will now be described in more detail in the following.

### Detailed description of the invention

### Definitions

Prior to discussing the present invention in further details, the following terms and conventions will first be defined:
The term **"3-hydroxybutyric acid"** refers to the compound bearing the CAS registry number CAS 300-85-6.

The term **"infant"** means a child under the age of 12 months (<12 month). The expression **"child"** means a child aged between one and eight years (≥1 year to <3 years), also called toddler. The expression **"child"** means a child between three and less than eight years of age ((≥3 year to <8 years).

An **"infant or young child born by C-section"** means an infant or young child who was delivered by caesarean. It means that the infant or young child was not vaginally delivered.

An **"infant or young child vaginally born"** means an infant or young child who was vaginally delivered and not delivered by caesarean.

A **"preterm" or "premature"** means an infant or young child who was not born at term. Generally it refers to an infant or young child born prior 37 weeks of gestation.

An **"infant having a low birth weight"** means a new born having a body weight below 2500g (5.5 pounds) either because of preterm birth or restricted fetal growth. It therefore encompasses:
- infant or young child who has/had a body weight from 1500 to 2500 g at birth (usually called "low birth weight" or LBW)
- infant or young child who has/had a body weight from 1000 to 1500 g at birth (called "very low birth weight" or VLBW)
- infant or young child who has/had a body weight under 1000 g at birth (called "extremely low birth weight" or ELBW).

An **"infant born small for gestational age (SGA)"** means a baby with birth weights below the 10^{th} percentile for babies of the same gestational age.

The expression **"nutritional composition"** means a composition which nourishes a subject. This nutritional composition is usually to be taken orally, and it usually includes a lipid or fat source and a protein source.

In a particular embodiment, the composition of the present invention is a hypoallergenic nutritional composition. The expression **"hypoallergenic nutritional composition"** means a nutritional composition which is unlikely to cause allergic reactions.

In a particular embodiment, the composition of the present invention is a "synthetic nutritional composition". The expression **"synthetic nutritional composition"** means a mixture obtained by chemical and/or biological means, which can be chemically identical to the mixture naturally occurring in mammalian milks (i.e. the synthetic composition is not breast milk).

The expression **"infant formula"** as used herein refers to a foodstuff intended for particular nutritional use by infants during the first months of life and satisfying by itself the nutritional requirements of this category of person (Article 2(c) of the European Commission Directive 91/321/EEC 2006/141/EC of 22 December 2006 on infant formulae and follow-on formulae). It also refers to a nutritional composition intended for infants and as defined in Codex Alimentarius (Codex STAN 72-1981) and Infant Specialities (incl. Food for Special Medical Purpose). The expression "infant formula" encompasses both "starter infant formula" and "follow-up formula" or "follow-on formula".

A **"follow-up formula"** or **"follow-on formula"** is given from the 6th month onwards. It constitutes the principal liquid element in the progressively diversified diet of this category of person.

The expression **"baby food"** means a foodstuff intended for particular nutritional use by infants or young children during the first years of life.

The expression **"infant cereal composition"** means a foodstuff intended for particular nutritional use by infants or young children during the first years of life.

The expression **"growing-up milk"** (or GUM) refers to a milk-based drink generally with added vitamins and minerals, that is intended for young children or children.

The term **"fortifier"** refers to liquid or solid nutritional compositions suitable for mixing with breast milk or infant formula.

The expression **"weaning period"** means the period during which the mother's milk is substituted by other food in the diet of an infant or young child.

The expressions **"days/weeks/months/years of life"** and **"days/weeks/months/years of birth"** can be used interchangeably.

The expression **"improved gastrointestinal barrier",** may encompass one or several of the following:
- Improved barrier repair, such as (but not limited to) recovery of the integrity of the gastrointestinal barrier, such as repair of a disrupted barrier, reduction of permeability upon inflammatory challenge of the gastrointestinal mucosa, and mucosal repair.
- Improved barrier structure, such as (but not limited to) strengthening of the gastrointestinal barrier, integrity of the gastrointestinal barrier, tight junction structure, and intestinal epithelial lining integrity.
- Improved barrier function, such as improvement of gastrointestinal barrier resistance, reduction of gastrointestinal barrier permeability, such as reduction of pathogens to migrate out of the gut through the intestinal barrier, such as reduction of commensal bacteria to migrate out of the gut through the intestinal barrier, reduction of allergens to migrate out of the gut through the intestinal barrier, reduction of toxic compounds to migrate out of the gut through the intestinal barrier and reduction of disease susceptibility.
- Improved barrier protection, such as (but not limited to) prevention of barrier dysfunction, prevention of barrier leakiness, protection of tight junction structure, protection of the intestinal epithelial lining integrity.

The **"mother's milk"** should be understood as the breast milk or the colostrum of the mother.

An **"oligosaccharide"** is a saccharide polymer containing a small number (typically three to ten) of simple sugars (monosaccharides).

The term **"HMO"** or **"HMOs"** or **"HMO's"** refers to human milk oligosaccharide(s). These carbohydrates are highly resistant to enzymatic hydrolysis, indicating that they may display essential functions not directly related to their caloric value. It has especially been illustrated that they play a vital role in the early development of infants and young children, such as the maturation of the immune system. Many different kinds of HMOs are found in the human milk. Each individual oligosaccharide is based on a combination of glucose, galactose, sialic acid (N-acetylneuraminic acid), fucose and/or N-acetylglucosamine with many and varied linkages between them, thus accounting for the enormous number of different oligosaccharides in human milk - over 130 such structures have been identified so far. Almost all of them have a lactose moiety at their reducing end while sialic acid and/or fucose (when present) occupy terminal positions at the non-reducing ends. The HMOs can be acidic (e.g. charged sialic acid containing oligosaccharide) or neutral (e.g. fucosylated oligosaccharide).

A **"fucosylated oligosaccharide"** is an oligosaccharide having a fucose residue. It has a neutral nature. Some examples are 2-FL (2'-fucosyllactose), 3-FL (3-fucosyllactose), difucosyllactose, lacto-N-fucopentaose (e.g. lacto-N-fucopentaose I, lacto-N-fucopentaose II, lacto-N-fucopentaose III, lacto-N-fucopentaose V), lacto-N-fucohexaose, lacto-N-difucohexaose I, fucosyllacto-N-hexaose, fucosyllacto-N-neohexaose, difucosyllacto-N-hexaose I, difucosyllacto-N-neohexaose II and any combination thereof. Without wishing to be bound by theory it is believed that the fucosyl- epitope of the fucosylated oligosaccharides may act as decoy at the mucosal surface. By a competition effect, it may prevent and/or limit the action of the pathogens responsible of infections (of viral or bacterial origin) or of their secreted components (e.g. toxins), especially by avoiding their binding to natural ligands, and without to be bound by theory, this is believed to therefore reduce the risk of infections/inflammations, and particularly the risk of LRT/ear infections and/or inflammations. In addition, the fucosylated oligosaccharides are thought to boost growth and metabolic activity of specific commensal microbes reducing inflammatory response and creating an environment unfavourable for pathogens thus leading to colonization resistance.

The expressions **"fucosylated oligosaccharides comprising a 2'-fucosyl-epitope"** and **"2-fucosylated oligosaccharides"** encompass fucosylated oligosaccharides with a certain homology of form since they contain a 2'-fucosyl-epitope, therefore a certain homology of function can be expected. Without wishing to be bound by theory the 2'- fucosyl-epitope of these fucosylated oligosaccharides is believed to be particularly specific to pathogens (or their secreted components) involved in the LRT and/or ear infections.

The expression **"N-acetylated oligosaccharide(s)"** encompasses both "N-acetyl-lactosamine" and "oligosaccharide(s) containing N-acetyl-lactosamine". They are neutral oligosaccharides having an N-acetyl-lactosamine residue. Suitable examples are LNT (lacto-N-tetraose), para-lacto-N-neohexaose (para-LNnH), LNnT (lacto-N-neotetraose) and any combinations thereof. Other examples are lacto-N-hexaose, lacto-N-neohexaose, para- lacto-N-hexaose, para-lacto-N-neohexaose, lacto-N-octaose, lacto-N- neooctaose, iso- lacto-N-octaose, para-lacto-N-octaose and lacto-N-decaose.

The expression "at least one fucosylated oligosaccharide" and "at least one N-acetylated oligosaccharide" means "at least one type of fucosylated oligosaccharide" and "at least one type of N-acetylated oligosaccharide".

A **"precursor of HMO"** is a key compound that intervenes in the manufacture of HMO, such as sialic acid and/or fucose.

A **"sialylated oligosaccharide"** is a charged sialic acid containing oligosaccharide, i.e. an oligosaccharide having a sialic acid residue. It has an acidic nature. Some examples are 3-SL (3' sialyllactose) and 6-SL (6' sialyllactose).

The nutritional composition of the present invention can be in solid form (e.g. powder) or in liquid form. The amount of the various ingredients (e.g. the oligosaccharides) can be expressed in **g/100g** of composition on a dry weight basis when it is in a solid form, e.g. a powder, or as a concentration in **g/L** of the composition when it refers to a liquid form (this latter also encompasses liquid composition that may be obtained from a powder after reconstitution in a liquid such as milk, water..., e.g. a reconstituted infant formula or a follow-on/follow-up formula or a growing-up milk or an infant cereal product or any other formulation designed for infant nutrition).

The term **"prebiotic"** means non-digestible carbohydrates that beneficially affect the host by selectively stimulating the growth and/or the activity of healthy bacteria such as bifidobacteria in the colon of humans (Gibson GR, Roberfroid MB. Dietary modulation of the human colonic microbiota: introducing the concept of prebiotics. J Nutr. 1995;125:1401-12).

The term **"probiotic"** means microbial cell preparations or components of microbial cells with a beneficial effect on the health or well-being of the host. (Salminen S, Ouwehand A. Benno Y. et al. "Probiotics: how should they be defined" Trends Food Sci. Technol. 1999: 10 107-10). The microbial cells are generally bacteria or yeasts.

The term **"cfu"** should be understood as colony-forming unit.

All percentages are by weight unless otherwise stated.

In addition, in the context of the invention, the terms "comprising" or "comprises" do not exclude other possible elements. The composition of the present invention, including the many embodiments described herein, can comprise, consist of, or consist essentially of the essential elements and limitations of the invention described herein, as well as any additional or optional ingredients, components, or limitations described herein or otherwise depending on the needs.

Any reference to prior art documents in this specification is not to be considered an admission that such prior art is widely known or forms part of the common general knowledge in the field.

The invention will now be described in further details. It is noted that the various aspects, features, examples and embodiments described in the present application may be compatible and/or combined together.

### Nutritional composition

As disclosed in the example section, it has been found that 3-hydroxybutyric acid is effective in improving the gastrointestinal barrier. Thus the invention relates to a nutritional composition comprising 3-hydroxybutyric acid for use in improving the gastrointestinal barrier in a subject wherein said use is selected from the groups consisting of improving the prevention of barrier dysfunction, the prevention of barrier leakiness, the protection of tight junction structure and the protection of the intestinal epithelial lining integrity; and/or
wherein the subject has an impaired microbiota; wherein 3-hydroxybutyric acid is present in an amount of 0.01 mg/L to 10 g/L of the composition, or wherein 3-hydroxybutyric acid is present in an amount of 0.007 mg/100 g to 7 g/100 g of the composition on a dry weight basis; or wherein 3-hydroxybutyric acid is present in an amount of 0.003 mg to 3 g per serving. In a preferred aspect, the subject is an infant (child under the age of 12 month) or a young child (between 1 year and less than 3 years). In another preferred aspect the nutritional composition is a growing-up milk and the subject is a child (aged from 3 years to less than 8 years).

In an embodiment, said improvement to the gastrointestinal barrier is improved barrier structure, improved barrier function improved barrier protection and/or improved barrier repair.

In another embodiment, said improvement to the gastrointestinal barrier is improved barrier function, improved barrier structure, and/or improved barrier protection.

In yet an embodiment, said improvement to the gastrointestinal barrier is improved barrier protection.

In the present invention, said use is for improving the prevention of barrier dysfunction, the prevention of barrier leakiness, the protection of tight junction structure and the protection of the intestinal epithelial lining integrity. In a further embodiment, said prevention of barrier leakiness is prevention of pathogens, allergens and/or toxic compounds to migrate from the gut into the body through the intestinal barrier.

The gastrointestinal barrier results in improved gastrointestinal barrier structure and function, as a gastrointestinal barrier missing proper protection would lose its optimal structure and function, namely upon inflammatory challenges.

Thus in an embodiment said use is for improving the strength of the gastrointestinal barrier, integrity of the gastrointestinal barrier, tight junction structure, and intestinal epithelial lining integrity.

In another embodiment, said use is for improving the gastrointestinal barrier resistance and for reducing gastrointestinal barrier permeability. Preferably said use is for reducing pathogens to migrate out of the gut through the intestinal barrier, such as reduction of commensal bacteria to migrate out of the gut through the intestinal barrier, reduction of allergens to migrate out of the gut through the intestinal barrier, reduction of toxic compounds to migrate out of the gut through the intestinal barrier, and reduction of disease susceptibility

The above outlined effects/benefits are preferably obtained in the small intestine.

In yet an embodiment, the nutritional composition is formulated for administration to an infant. In another embodiment, said infant is selected from the group consisting of premature, small for gestational age and low birth weight babies, preferably the infant is premature. The nutritional compositions according to the invention are considered particularly useful for these types of infants, since the described benefits are more important for these infants than for "normal" infants, because the barrier is less mature and more permeable than in the healthy term infant.

In one alternative of the present invention, 3-hydroxybutyric acid is in an amount of 0.01 mg/L to 10 g/L of the composition, such as 0.1 mg/L to 1 g/L, or 0.5 mg/L to 500 mg/L of the composition. In a particular embodiment, 3-hydroxybutyric acid is in an amount of 1mg/L of the composition. Such concentrations are typically used when the nutritional composition is in the form of a complete nutrition, including diverse nutrients such as proteins, fats and carbohydrates. In a particular embodiment, 3-hydroxybutyric acid is in an amount of 33 mg/L of the composition.

Such concentrations are typically used when the nutritional composition is in the form of a nutritional supplement or fortifier.

In another alternative of the present invention, 3-hydroxybutyric acid is in an amount of 0.007 mg/100g to 7 g/100g of the composition on a dry weight basis, such as 0.07 mg/100g to 0.7 g/100g or 0.3 mg/100g to 0.4 g/100g of the composition on a dry weight basis. In a particular embodiment, 3-hydroxybutyric acid is in an amount of 1 mg/100g of the composition on a dry weight basis. Such concentrations are typically used when the nutritional composition is in the form of a complete nutrition, including diverse nutrients such as proteins, fats and carbohydrates.

In another alternative of the present invention, 3-hydroxybutyric acid is in an amount of 0.003 mg to 3 g per serving, such as 0.03 mg to 0.3 g or 0.15 mg to 0.15 g per serving. In a particular embodiment, 3-hydroxybutyric acid is in an amount of 0.3 mg per serving. Such concentrations are preferably used when the nutritional composition is in the form of a nutritional supplement or fortifier.

In a particular embodiment, 3-hydroxybutyric acid is provided in the nutritional composition of the present invention in such an amount that normal consumption of the nutritional composition would provide to the subject consuming it, preferably the infant or young child, respectively the child, consuming it a total daily dose of 0.006 mg to 6 g, such as 0.06 mg to 0.6 g or 0.3 mg to 0.3 g per day. It is believed that a minimal amount of 3-hydroxybutyric acid is necessary to have the desired effect in a measurable way.

The nutritional composition according to the present invention generally contains a carbohydrate source. Any source of carbohydrate can be used, such as lactose, sucrose, saccharose, maltodextrin, starch and mixtures thereof although one of the preferred sources of carbohydrates is lactose.

The nutritional composition according to the present invention may also comprise oligosaccharides. Preferably the composition comprises oligosaccharide(s) (i.e. other than human milk oligosaccharides mentioned above) and/or at least a fiber(s) and/or at least a precursor(s) thereof. The other oligosaccharide and/or fiber and/or precursor thereof may be selected from the list comprising galactooligosaccharides (GOS), fructo-oligosaccharides (FOS), inulin, xylooligosaccharides (XOS), polydextrose, human milk oligosaccharides (as defined in the definition section), bovine milk oligosaccharides and any combination thereof. They may be in an amount between 0 and 10% by weight of composition.

Suitable commercial products that can be used to prepare the nutritional compositions according to the invention include combinations of FOS with inulin such as the product sold by BENEO under the trademark Orafti, or polydextrose sold by Tate & Lyle under the trademark STA-LITE^{®}.

The nutritional composition according to the present invention may optionally also comprise at least one precursor of oligosaccharide. There can be one or several precursor(s) of oligosaccharide. For example the precursor of human milk oligosaccharide is sialic acid, fucose or a mixture thereof. In some particular embodiments the composition comprises sialic acid.

In particular examples the nutritional composition comprises from 0 to 3 g/L of precursor(s) of oligosaccharide, or from 0 to 2 g/L, or from 0 to 1 g/L, or from 0 to 0.7 g/L, or from 0 to 0.5 g/L or from 0 to 0.3 g/L, or from 0 to 0.2 g/L of precursor(s) of oligosaccharide. The composition according to the invention can contain from 0 to 2.1 g of precursor(s) of oligosaccharide per 100g of composition on a dry weight basis, e.g. from 0 to 1.5 g or from 0 to 0.8 g or from 0 to 0.15 g of precursor(s) of oligosaccharide per 100g of composition on a dry weight basis.

The nutritional composition of the present invention can further comprise at least one probiotic (or probiotic strain), such as a probiotic bacterial strain.

The probiotic microorganisms most commonly used are principally bacteria and yeasts of the following genera: *Lactobacillus spp., Streptococcus spp., Enterococcus spp., Bifidobacterium spp.* and *Saccharomyces spp.*

In some particular embodiments, the probiotic is a probiotic bacterial strain. In some specific embodiments, it is particularly *Bifidobacteria* and/or *Lactobacilli.*

Suitable probiotic bacterial strains include *Lactobacillus rhamnosus* ATCC 53103 available from Valio Oy of Finland under the trademark LGG, *Lactobacillus rhamnosus* CGMCC 1.3724, *Lactobacillus paracasei* CNCM I-2116, *Lactobacillus johnsonii* CNCM I-1225, *Streptococcus salivarius* DSM 13084 sold by BLIS Technologies Limited of New Zealand under the designation KI2, *Bifidobacterium lactis* CNCM 1-3446 sold *inter alia* by the Christian Hansen company of Denmark under the trademark Bb 12, *Bifidobacterium longum* ATCC BAA-999 sold by Morinaga Milk Industry Co. Ltd. of Japan under the trademark BB536, *Bifidobacterium breve* sold by Danisco under the trademark Bb-03, *Bifidobacterium breve* sold by Morinaga under the trade mark M-16V, *Bifidobacterium infantis* sold by Procter & GambIe Co. under the trademark Bifantis and *Bifidobacterium breve* sold by Institut Rosell (Lallemand) under the trademark R0070.

The nutritional composition according to the invention may contain from 10E3 to 10E12 cfu of probiotic strain, more preferably between 10E7 and 10E12 cfu such as between 10E8 and 10E10 cfu of probiotic strain per g of composition on a dry weight basis.

In one embodiment the probiotics are viable. In another embodiment the probiotics are non-replicating or inactivated. There may be both viable probiotics and inactivated probiotics in some other embodiments. Probiotic components and metabolites can also be added.

In one embodiment, the nutritional composition of the invention is a complete nutritional composition (fulfilling all or most of the nutritional needs of the subject). In another embodiment the nutrition composition is a supplement or a fortifier intended for example to supplement human milk or to supplement an infant formula or a follow-on/follow-up formula.

In some particular embodiments, the composition of the invention is an infant formula, a fortifier or a supplement that may be intended for the first 4, 6 or 12 months of age. In a preferred embodiment the nutritional composition of the invention is an infant formula. It is indeed believed that the nutritional intervention of the invention may be most effective when enacted at an early stage of life (for example the first 1, 4, 6, 12 months of age).

The nutritional composition according to the invention can be for example an infant formula, a starter infant formula, a follow-on or follow-up formula, a growing-up milk, a baby food, an infant cereal composition, a fortifier such as a human milk fortifier, a supplement, a healthcare product, a medical food, a tube feed composition or an animal feed.

In some particular embodiments, the composition of the invention is an infant formula, a fortifier or a supplement that may be intended for the first 4 or 6 months of age. In a preferred embodiment the nutritional composition of the invention is an infant formula.

In some other embodiments the nutritional composition of the present invention is a fortifier. The fortifier can be a breast milk fortifier (e.g. a human milk fortifier) or a formula fortifier such as an infant formula fortifier or a follow-on/follow-up formula fortifier.

When the nutritional composition is a supplement, it can be provided in the form of unit doses. In such cases it is particularly useful to define the amount of 3-hydroxybutyric acid in terms or daily dose to be administered to the infant or young child, such as described above.

The nutritional composition of the present invention can be in solid (e.g. powder), liquid or gelatinous form.

In a specific embodiment the nutritional composition is a supplement in powder form and provided in a sachet, in the form of tablets, capsules, pastilles or a liquid, such as a liquid to be dispensed as drops in breast milk or in a nutritional composition or directly in the mouth of an infant or a young child.

In another embodiment, the supplement may further contain a carrier, protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents and gel forming agents. The supplement may also contain conventional pharmaceutical additives and adjuvants, excipients and diluents, including, but not limited to, water, gelatine of any origin, vegetable gums, lignin-sulfonate, talc, sugars, starch, gum arabic, vegetable oils, polyalkylene glycols, flavouring agents, preservatives, stabilizers, emulsifying agents, buffers, lubricants, colorants, wetting agents, and fillers. When the supplement is in powder form, it may comprise a carrier. It is however preferred that the supplement is devoid of a carrier. When the supplement is in the form of a syrup, the HMOs are preferably dissolved or suspended in water acidified with citrate.

Further, the supplement may contain vitamins, minerals trace elements and other micronutrients in accordance with the recommendations of Government bodies such as the USRDA.

The nutritional composition of the present invention can be in solid (e.g. powder), liquid or gelatinous form. In a specific embodiment the nutritional composition is a supplement comprising 3-hydroxybutyric acid, wherein the supplement is in powder form and provided in a sachet or in the form of a syrup, preferably a syrup with a total solid concentration of 5 to 75 g/100 mL (5 to 75% (w/v)). When the supplement is in powder form, it may comprise a carrier. It is however preferred that the supplement is devoid of a carrier. When the supplement is in the form of a syrup, 3-hydroxybutyric acid is preferably dissolved or suspended in water acidified with citrate.

The nutritional composition to the invention generally contains a protein source. The protein can be in an amount of from 1.6 to 3 g per 100 kcal. In some embodiments, especially when the composition is intended for premature infants, the protein amount can be between 2.4 and 4 g/100kcal or more than 3.6 g/100kcal. In some other embodiments the protein amount can be below 2.0 g per 100 kcal, e.g. between 1.8 to 2 g/100kcal, or in an amount below 1.8g per 100 kcal.

The type of protein is not believed to be critical to the present invention provided that the minimum requirements for essential amino acid content are met and satisfactory growth is ensured. Thus, protein sources based on whey, casein and mixtures thereof may be used as well as protein sources based on soy. As far as whey proteins are concerned, the protein source may be based on acid whey or sweet whey or mixtures thereof and may include alpha-lactalbumin and betalactoglobulin in any desired proportions.

In some advantageous embodiments, the protein source is whey predominant (i.e. more than 50% of proteins are coming from whey proteins, such as 60% or 70%).

The proteins may be intact or hydrolysed or a mixture of intact and hydrolysed proteins. By the term "intact" is meant that the main part of the proteins are intact, i.e. the molecular structure is not altered, for example at least 80% of the proteins are not altered, such as at least 85% of the proteins are not altered, preferably at least 90% of the proteins are not altered, even more preferably at least 95% of the proteins are not altered, such as at least 98% of the proteins are not altered. In a particular embodiment, 100% of the proteins are not altered.

The term "hydrolysed" means in the context of the present invention a protein which has been hydrolysed or broken down into its component amino acids. The proteins may be either fully or partially hydrolysed. It may be desirable to supply partially hydrolysed proteins (degree of hydrolysis between 2 and 20%), for example for infants or young children believed to be at risk of developing cow's milk allergy. If hydrolysed proteins are required, the hydrolysis process may be carried out as desired and as is known in the art. For example, whey protein hydrolysates may be prepared by enzymatically hydrolysing the whey fraction in one or more steps. If the whey fraction used as the starting material is substantially lactose free, it is found that the protein suffers much less lysine blockage during the hydrolysis process. This enables the extent of lysine blockage to be reduced from about 15% by weight of total lysine to less than about 10% by weight of lysine; for example about 7% by weight of lysine which greatly improves the nutritional quality of the protein source.

In an embodiment of the invention at least 70% of the proteins are hydrolysed, preferably at least 80% of the proteins are hydrolysed, such as at least 85% of the proteins are hydrolysed, even more preferably at least 90% of the proteins are hydrolysed, such as at least 95% of the proteins are hydrolysed, particularly at least 98% of the proteins are hydrolysed. In a particular embodiment, 100% of the proteins are hydrolysed.

In one particular embodiment the proteins of the nutritional composition are hydrolyzed, fully hydrolyzed or partially hydrolyzed. The degree of hydrolysis (DH) of the protein can be between 8 and 40, or between 20 and 60 or between 20 and 80 or more than 10, 20, 40, 60, 80 or 90.

The protein component can alternatively be replaced by a mixture or synthetic amino acid, for example for preterm or low birth weight infants.

In a particular embodiment the nutritional composition according to the invention is a hypoallergenic composition. In another particular embodiment the composition according to the invention is a hypoallergenic nutritional composition.

The nutritional composition according to the present invention generally contains a source of lipids. This is particularly relevant if the nutritional composition of the invention is an infant formula. In this case, the lipid source may be any lipid or fat, preferably a source which is suitable for use in infant formulae. Some suitable fat sources include palm oil, structured triglyceride oil, high oleic sunflower oil and high oleic safflower oil, medium-chain-triglyceride oil. The essential fatty acids linoleic and α-linolenic acid may also be added, as well small amounts of oils containing high quantities of preformed arachidonic acid and docosahexaenoic acid such as fish oils or microbial oils. The fat source may have a ratio of n-6 to n-3 fatty acids of about 5:1 to about 15:1; for example about 8:1 to about 10:1.

The nutritional composition of the invention may also contain all vitamins and minerals understood to be essential in the daily diet and in nutritionally significant amounts. Minimum requirements have been established for certain vitamins and minerals. Examples of minerals, vitamins and other nutrients optionally present in the composition of the invention include vitamin A, vitamin B1, vitamin B2, vitamin B6, vitamin B12, vitamin E, vitamin K, vitamin C, vitamin D, folic acid, inositol, niacin, biotin, pantothenic acid, choline, calcium, phosphorous, iodine, iron, magnesium, copper, zinc, manganese, chlorine, potassium, sodium, selenium, chromium, molybdenum, taurine, and L-carnitine. Minerals are usually added in salt form. The presence and amounts of specific minerals and other vitamins will vary depending on the intended population.

If necessary, the nutritional composition of the invention may contain emulsifiers and stabilisers such as soy, lecithin, and citric acid esters of mono- and diglycerides.

The nutritional composition of the invention may also contain other substances which may have a beneficial effect such as lactoferrin, nucleotides, and nucleosides.

The nutritional composition of the invention may also contain carotenoid(s). In some particular embodiments of the invention, the nutritional composition of the invention does not comprise any carotenoid.

The nutritional composition according to the invention may be prepared in any suitable manner. A composition will now be described by way of example.

For example, a nutritional composition such as an infant formula may be prepared by blending together the protein source, the carbohydrate source and the fat source in appropriate proportions. If used, the emulsifiers may be included at this point. The vitamins and minerals may be added at this point but they are usually added later to avoid thermal degradation. Any lipophilic vitamins, and emulsifiers may be dissolved into the fat source prior to blending. Water, preferably water which has been subjected to reverse osmosis, may then be mixed in to form a liquid mixture. The temperature of the water is conveniently in the range between about 50°C and about 80°C to aid dispersal of the ingredients. Commercially available liquefiers may be used to form the liquid mixture.

The fucosylated oligosaccharide(s) and the N-acetylated oligosaccharide(s) may be added at this stage, especially if the final product is to have a liquid form. If the final product is to be a powder, they may likewise be added at this stage if desired.

The liquid mixture is then homogenised, for example in two stages.

The liquid mixture may then be thermally treated to reduce bacterial loads, by rapidly heating the liquid mixture to a temperature in the range between about 80°C and about 150°C for a duration between about 5 seconds and about 5 minutes, for example. This may be carried out by means of steam injection, an autoclave or a heat exchanger, for example a plate heat exchanger.

Then, the liquid mixture may be cooled to between about 60°C and about 85°C for example by flash cooling. The liquid mixture may then be again homogenised, for example in two stages between about 10 MPa and about 30 MPa in the first stage and between about 2 MPa and about 10 MPa in the second stage. The homogenised mixture may then be further cooled to add any heat sensitive components, such as vitamins and minerals. The pH and solids content of the homogenised mixture are conveniently adjusted at this point.

If the final product is to be a powder, the homogenised mixture is transferred to a suitable drying apparatus such as a spray dryer or freeze dryer and converted to powder. The powder should have a moisture content of less than about 5% by weight. The fucosylated oligosaccharide(s) and the N-acetylated oligosaccharide(s) may also or alternatively be added at this stage by dry-mixing or by blending them in a syrup form of crystals, along with the probiotic strain(s) (if used), and the mixture is spray-dried or freeze-dried.

If a liquid composition is preferred, the homogenised mixture may be sterilised then aseptically filled into suitable containers or may be first filled into the containers and then retorted.

In another embodiment, the composition of the invention may be a supplement. The supplement may be in the form of tablets, capsules, pastilles or a liquid for example. The supplement may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents and gel forming agents. The supplement may also contain conventional pharmaceutical additives and adjuvants, excipients and diluents, including, but not limited to, water, gelatine of any origin, vegetable gums, lignin-sulfonate, talc, sugars, starch, gum arabic, vegetable oils, polyalkylene glycols, flavouring agents, preservatives, stabilizers, emulsifying agents, buffers, lubricants, colorants, wetting agents, and fillers.

Further, the supplement may contain an organic or inorganic carrier material suitable for oral or parenteral administration as well as vitamins, minerals trace elements and other micronutrients in accordance with the recommendations of Government bodies such as the USRDA.

The nutritional composition according to the invention is for use in any type of human subject, preferably in infants or young children. The infants or young children may be born term or preterm. In a particular embodiment the nutritional composition of the invention is for use in infants or young children that were born preterm, having a low birth weight and/or born small for gestational age (SGA). In a particular embodiment the nutritional composition of the invention is for use in preterm infants, infants having a low birth weight and/or infants born small for gestational age (SGA).

The nutritional composition of the present invention may also be used in an infant or a young child that was born by C-section or that was vaginally delivered.

In some embodiments the composition according to the invention can be for use before and/or during the weaning period.

The nutritional composition can be administered (or given or fed) at an age and for a period that depends on the needs.

The nutritional composition can be for example given immediately after birth of the infants. The composition of the invention can also be given during the first week of life of the infant, or during the first 2 weeks of life, or during the first 3 weeks of life, or during the first month of life, or during the first 2 months of life, or during the first 3 months of life, or during the first 4 months of life, or during the first 6 months of life, or during the first 8 months of life, or during the first 10 months of life, or during the first year of life, or during the first two years of life or even more. In some particularly advantageous embodiments of the invention, the nutritional composition is given (or administered) to an infant within the first 4, 6 or 12 months of birth of said infant. In some other embodiments, the nutritional composition of the invention is given few days (e.g. 1, 2, 3, 5, 10, 15, 20...), or few weeks (e.g. 1, 2, 3, 4, 5, 6, 7, 8, 9, 10...), or few months (e.g. 1, 2, 3, 4, 5, 6, 7, 8, 9, 10...) after birth. This may be especially the case when the infant is premature, but not necessarily.

In one embodiment the composition of the invention is given to the infant or young child as a supplementary composition to the mother's milk. In some embodiments the infant or young child receives the mother's milk during at least the first 2 weeks, first 1, 2, 4, or 6 months. In one embodiment the nutritional composition of the invention is given to the infant or young child after such period of mother's nutrition, or is given together with such period of mother's milk nutrition. In another embodiment the composition is given to the infant or young child as the sole or primary nutritional composition during at least one period of time, e.g. after the 1^{st}, 2^{nd} or 4^{th} month of life, during at least 1, 2, 4 or 6 months.

In one embodiment the nutritional composition of the invention is a complete nutritional composition (fulfilling all or most of the nutritional needs of the subject). In another embodiment the nutrition composition is a supplement or a fortifier intended for example to supplement human milk or to supplement an infant formula or a follow-on/follow-up formula.

The invention will now be described in further details in the following non-limiting examples.

### Examples

### Example 1

An example of the composition of a nutritional composition (e.g. an infant formula) according to the present invention is given in the below table 1. This composition is given by way of illustration only.

**Table 1: Composition of the infant formula of Example 1**

| **Nutrients** | **per 100kcal** | **per litre** |
|---|---|---|
| Energy (kcal) | 100 | 670 |
| Protein (g) | 1.83 | 12.3 |
| Fat (g) | 5.3 | 35.7 |
| Linoleic acid (g) | 0.79 | 5.3 |
| α-Linolenic acid (mg) | 101 | 675 |
| Lactose (g) | 11.2 | 74.7 |
| Minerals (g) | 0.37 | 2.5 |
| Na (mg) | 23 | 150 |
| K (mg) | 89 | 590 |
| Cl (mg) | 64 | 430 |
| Ca (mg) | 62 | 410 |
| P (mg) | 31 | 210 |
| Mg (mg) | 7 | 50 |
| Mn (µg) | 8 | 50 |
| Se (µg) | 2 | 13 |
| Vitamin A (µg RE) | 105 | 700 |
| Vitamin D (µg) | 1.5 | 10 |
| Vitamin E (mg TE) | 0.8 | 5.4 |
| Vitamin K1 (µg) | 8 | 54 |
| Vitamin C (mg) | 10 | 67 |
| Vitamin B1 (mg) | 0.07 | 0.47 |
| Vitamin B2 (mg) | 0.15 | 1.0 |
| Niacin (mg) | 1 | 6.7 |
| Vitamin B6 (mg) | 0.075 | 0.50 |
| Folic acid (µg) | 9 | 60 |
| Pantothenic acid (mg) | 0.45 | 3 |
| Vitamin B12 (µg) | 0.3 | 2 |
| Biotin (µg) | 2.2 | 15 |
| Choline (mg) | 10 | 67 |
| Fe (mg) | 1.2 | 8 |
| I (µg) | 15 | 100 |
| Cu (mg) | 0.06 | 0.4 |
| Zn (mg) | 0.75 | 5 |
| 3-hydroxybutyric acid (mg) | 0.15 | 1 |

### Example 2 -Correlation of HMO metabolites with effect of HMOs on gut barrier function

### Aim of study

To correlate the effect of the microbiota fermentation products (i.e. metabolites) of 2'FL, 2'FL+LNnT and combination of 6 HMOs (2'FL, 3'SL, 6'SL, LNT, LNnT and DiFL) with respect to their ability to protect and strengthen the epithelial barrier before and after inflammatory challenge in a co-culture cell-line model of intestinal epithelial cells containing Caco-2 and HT29-MTX cells with the respective amounts of individual metabolites identified in the microbiota fermentation products of each HMO or mix of HMO.

### Methods

Microbiota fermentation products of 2'FL, 2'FL+LNnT and combinations of 6 HMO (2'FL, 3'SL, 6'SL, LNT, LNnT and DiFL) were obtained by culturing a three-month old fecal breast-fed baby microbiota in a continuous model of the human gastrointestinal tract called Simulator of the Human Intestinal Microbial Ecosystem (SHIME) obtained from ProDigest, Gent, Belgium. Four separate SHIME culture vessels were inoculated with the same baby microbiota. After 2 weeks of microbiota stabilization, the microbiota SHIME vessels were fed with either 2'FL, a combination of 2'FL and LNnT (2:1 ratio) or a combination of 6 HMOs (2'FL, 3'SL, 6'SL, LNT, LNnT and DiFL) every day at a concentration of 5g/L for 21 days. The ratios of the different HMOs in the 6 HMO bend were as follows: 2'FL : 3'SL : 6'SL : LNnT : LNT : DiFL = 0.55: 0.07 : 0.09 : 0.05 : 0.18 : 0.06. Fermented culture media on each microbiota vessel were collected before the first feeding and then 2 and 21 days after the first feeding. All the fermented culture media were then centrifuged and the supernatants comprising the metabolites of the HMOs were collected.

For epithelial barrier studies, the human Caco-2 and HT29-MTX cell lines were obtained from the American Type Culture Collection (ATCC) and the European Collection of Authenticated Cell Cultures (ECACC), respectively. Caco-2 cell and HT29-MTX were maintained separately in culture flasks at 37°C under humidified 10% CO₂ atmosphere in Dulbecco's minimum essential media (DMEM) supplemented with GlutaMAX (Invitrogen), 1% minimum essential media, 100 µg/ml streptomycin, 100 UI/ml penicillin and heat inactivated fetal bovine serum (FBS; 15% for Caco-2 and 10% for HT29-MTX).

To make co-culture for epithelial barrier studies, each cell lines were expanded in their respective flasks until 90% confluent monolayers were reached. Cell lines are then trypsinized with 1x trypsin. Co-culture of Caco-2 and HT29-MTX were seeded at 6 x 10⁴/cm² on 1.12 cm² Transwell Polycarbonate semi-permeable membranes (0.4 µm) and grown in DMEM supplemented with GlutaMAX, 1% minimum essential media, 100 µg/ml streptomycin, 100 UI/ml penicillin and 10% heat inactivated FBS for 21 days.

On the day of the experiment, medium was replaced by fresh medium at least 4 hours before treatment. Co-cultures were first pre-treated with fermented media collected from the SHIME experiments. Specifically, fermented media from baby microbiota fed with 2'FL, 2'FL-LNnT or combination of 6 HMOs (HMO6) (2'FL, 3'SL, 6'SL, LNT, LNnT and DiFL) were added at the apical compartment of the transwell at a concentration of 20% v/v. Unfermented culture SHIME media (no baby microbiota) was used as control. After 36 hours of pre-treatment, epithelial barrier dysfunction was induced by adding TNF-α (2.5 ng/ml) and IFN-γ (10 ng/ml) at the basolateral compartment of the transwell for additional 48 hours. During the course of the experiment, transepithelial electrical resistance was continuously measured using a Cellzscope machine. At the end of the experiment, translocation of the FITC-labeled dextran (4000 Da) from the apical to basolateral compartment was quantified within a 2-hour period.

A metabolomic analysis of the microbiota fermentation products of 2'FL, 2'FL+LNnT and combinations of 6 HMO (2'FL, 3'SL, 6'SL, LNT, LNnT and DiFL) obtained with the SHIME was performed. Metabolomic analysis was performed using the Metabolon platform (Metabolon Inc, Morrisville, NC, USA). In brief, samples were extracted and split into equal parts for analysis on the LC/MS/MS and Polar LC platforms. Proprietary software was used to match ions to an inhouse library of standards for metabolite identification and for metabolite quantitation by peak area integration. All datasets were provided in electronic form to Nestle and analysed.

Statistical analysis was performed on the metabolomic data to correlate the level of efficiency of each microbiota fermentation products on the different barrier read-outs with the concentration of each metabolite in such microbiota fermentation product. The statistical analysis was as follows.

For epithelial barrier studies, differences between treatments were assessed using one-way analysis of variance and subsequent multiple comparisons using Fisher's least significant difference procedure with statistical significance level set at α=5% (LSD5%).

For the metabolomic correlation analysis, a three-step analysis was performed:
- All samples regardless of treatment was first divided into 2 groups:
   samples before feeding (day 0) and samples after feeding (day 2 and 21). Metabolites that significantly change between day 0 and day 21 were assessed using one-way analysis of variance and subsequent multiple comparisons using Fisher's least significant difference procedure with LSD5%.
- Significant metabolites were then clustered based on treatments.
   Metabolites that clustered more with HMO treatments compared to lactose were chosen to be used for correlation with the epithelial barrier studies

Correlation between metabolites quantity and epithelial barrier data was performed using Pearson correlation coefficient

### Results

Figure 1 shows the efficacy of HMOs fermentation products to provide prophylactic epithelial barrier protection whereas lactose fermentation, the major carbohydrate in human milk and infant formula do not.

Figure 2 shows the efficacy of HMOs fermentation products to induce resistance against inflammation-induced epithelial barrier dysfunction.

Figure 3 shows the efficacy of HMOs fermentation products to limit susceptibility to inflammation-induced epithelial barrier dysfunction.

Figure 4 shows the efficacy of HMOs fermentation products to reduce symptoms severity of inflammation-induced epithelial barrier dysfunction.

Figure 5 shows the efficacy of HMOs fermentation products to reduce symptoms severity of inflammation-induced epithelial barrier dysfunction.

Table 3 shows the correlation between the abundance of 3-hydroxybutyric acid in the HMO fermentation products and the different read-outs of epithelial barrier integrity. It shows that 3-hydroxybutyric acid has a significantly positive correlation with all the barrier integrity readouts.

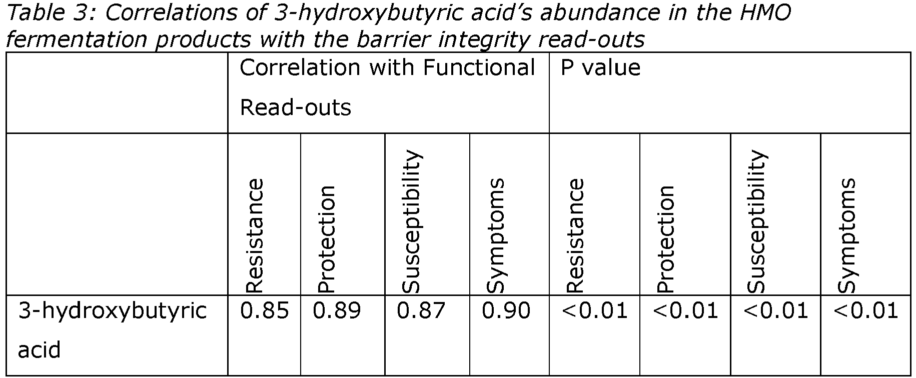

### Conclusion

These data show that 3-hydroxybutyric acid is efficient in improving and protecting the intestinal barrier.

### Example 3 - Direct effect of 3-hydroxybutyric acid

### Aim of study

3-Hydroxybutyric acid was tested with respect to its ability to protect and strengthen the epithelial barrier after inflammatory challenge in a co-culture cell-line model of intestinal epithelial cells containing Caco-2 and HT29-MTX cells.

### Materials and methods

The human Caco-2 and HT29-MTX cell lines were obtained from the American Type Culture Collection (ATCC) and the European Collection of Authenticated Cell Cultures (ECACC), respectively. Caco-2 cell and HT29-MTX were maintained separately in culture flasks at 37°C under humidified 10% CO₂ atmosphere in Dulbecco's minimum essential media (DMEM) supplemented with GlutaMAX (Invitrogen), 1% minimum essential media, 100 µg/ml streptomycin, 100 UI/ml penicillin and heat inactivated fetal bovine serum (FBS; 15% for Caco-2 and 10% for HT29-MTX).

To make co-culture for epithelial barrier studies, each cell lines were expanded in their respective flasks until 90% confluent monolayers were reached. Cell lines are then trypsinized with 1x trypsin. Co-culture of Caco-2 and HT29-MTX were seeded at 6 x 10⁴/cm² on 1.12 cm² Transwell Polycarbonate semi-permeable membranes (0.4 µm) and grown in DMEM supplemented with GlutaMAX, 1% minimum essential media, 100 µg/ml streptomycin, 100 UI/ml penicillin and 10% heat inactivated FBS for 21 days.

On the day of the experiment, medium was replaced by fresh medium at least 4 hours before treatment. Co-cultures were first pre-treated with 3-hydroxybutyric acid. Specifically, a 3-hydroxybutyric acid (Sigma 166898; CAS 300-85-6) stock solution was made by diluting the chemical in DMSO at a final concentration of 1 mM. On the day of experiment, stock solution was further diluted with fresh media at concentration of 100 µM. Diluted 3-hydroxybutyric acid was then added at the apical compartment of the transwell at a final concentration of 10 µM (eg 50 µl in 500 µl of apical compartment volume). For control, a volume of DMSO similar to stock 3-hydroxybutyric acid was diluted with fresh culture media and diluted DMSO was added at the apical compartment of the transwell at a concentration of 20% v/v. Unfermented culture SHIME media (no baby microbiota) was used as control. After 36 hours of pre-treatment, epithelial barrier dysfunction was induced by adding TNF-α (2.5 ng/ml) and IFN-γ (10 ng/ml) at the basolateral compartment of the transwell for additional 48 hours. At the end of the experiment, translocation of the FITC-labeled dextran (4000 Da) from the apical to basolateral compartment was quantified within a 2-hour period. FD4 translocation data were analysed by using the two-sided two-sample Student t-test assuming unequal variances.

### Results

Figure 6 shows the efficacy of 3-hydroxybutyric acid to protect the barrier against inflammation-induced epithelial barrier hyperpermeability.

### Conclusion

The presented data confirms the results of the correlation study and provides evidence of the efficacy of 3-hydroxybutyric acid in improving and protecting the intestinal barrier.

## Claims

1. A nutritional composition comprising 3-hydroxybutyric acid for use in improving the gastrointestinal barrier in a subject;
wherein said use is selected from the group consisting of improving the prevention of barrier dysfunction, the prevention of barrier leakiness, the protection of tight junction structure and the protection of the intestinal epithelial lining integrity; and/or
wherein the subject has an impaired microbiota;
wherein 3-hydroxybutyric acid is present in an amount of 0.01 mg/L to 10 g/L of the composition, or
wherein 3-hydroxybutyric acid is present in an amount of 0.007 mg/100 g to 7 g/100 g of the composition on a dry weight basis; or
wherein 3-hydroxybutyric acid is present in an amount of 0.003 mg to 3 g per serving.

2. A nutritional composition for use according to claim 1 wherein the subject is an infant (child under the age of 12 month) or a young child (between 1 year and less than 3 years).

3. A nutritional composition for use according to claim 1, wherein the nutritional composition is a growing-up milk and wherein the subject is a child aged from 3 years to less than 8 years.

4. The nutritional composition for use according to any one of the preceding claims, wherein said improvement to the gastrointestinal barrier is improved barrier structure, improved barrier function, improved barrier protection and/or improved barrier repair.

5. The nutritional composition for use according to any one of the preceding claims, wherein said improvement to the gastrointestinal barrier is improved barrier function, improved barrier structure, and/or improved barrier protection.

6. The nutritional composition for use according to any of the preceding claims, wherein said improvement to the gastrointestinal barrier is improved barrier protection.

7. The nutritional composition for use according to claim 1, wherein said use is for the prevention of barrier leakiness and wherein said prevention of barrier leakiness is prevention of pathogens, allergens and/or toxic compounds to migrate from the gut into the body through the intestinal barrier.

8. The nutritional composition for use according to claim 1, wherein the subject has an impaired microbiota and wherein the impaired microbiota is an unbalanced microbiota having abnormally low proportion of a *Bifidobacterium,* preferably abnormally low proportion of *Bifidobacterium longum* and/or Bifidobacterium bifidum, more preferably abnormally low proportion of *Bifidobacterium bifidum and*/*or Bifidobacterium longum* subsp. *infantis.*

9. The nutritional composition for use according to claim 1 or 8, wherein the impaired microbiota has less than 80%, preferably less than 70%, more preferably less than 60% of Actinobacteriaceae and even more preferably of Bifidobacterium, the percentages being defined by number based on total bacteria in the microbiota.

10. The nutritional composition for use according to any of claims 8 to 9, wherein the impaired microbiota has less than 20%, preferably less than 15%, more preferably less than 10% of *Bifidobacterium bifidum,* the percentages being defined by number based on total bacteria in the microbiota.

11. The nutritional composition for use according to any one of the preceding claims, wherein the subject is an infant selected from the group consisting of premature infants, small for gestational age infants and low birth weight infants, preferably a premature infant.

12. The nutritional composition for use according to any one of the preceding claims, wherein 3-hydroxybutyric acid is present in an amount of 0.1 mg/L to 1 g/L of the composition or in an amount of 0.07 mg/100 g to 0.7 g/100 g of the composition on a dry weight basis; and/or wherein 3-hydroxybutyric acid is present in an amount of 0.03 mg to 0.3 g per serving.

13. The nutritional composition for use according to any one of the preceding claims, wherein 3-hydroxybutyric acid is provided in such an amount that normal consumption of the nutritional composition would provide to the subject consuming it a total daily dose of 0.006 mg to 6 g per day.

14. The nutritional composition for use according to any one of claims 1 and 4 to 13, wherein said nutritional composition is an infant formula, a starter infant formula, a follow-on or follow-up formula, a growing-up milk, a baby food, an infant cereal composition, a fortifier such as a human milk fortifier, a supplement, a healthcare product, a medical food or a tube feed composition.

15. The nutritional composition for use according to claim 2, wherein said nutritional composition is an infant formula, a starter infant formula, a follow-on or follow-up formula, a growing-up milk, a baby food, an infant cereal composition, a fortifier such as a human milk fortifier or a supplement.

## Patentansprüche

1. Nährstoffzusammensetzung, umfassend 3-Hydroxybuttersäure, zur Verwendung bei einem Verbessern der gastrointestinalen Barriere bei einem Subjekt;
wobei die Verwendung aus der Gruppe ausgewählt ist, bestehend aus dem Verbessern der Prävention einer Barrieredysfunktion, der Prävention einer Barrieredurchlässigkeit, dem Schutz einer Tight Junction-Struktur und dem Schutz der Darmepithelschleimhautintegrität; und/oder
wobei das Subjekt eine beeinträchtigte Mikrobiota aufweist;
wobei 3-Hydroxybuttersäure in einer Menge von 0,01 mg/l bis 10 g/l der Zusammensetzung vorhanden ist oder
wobei 3-Hydroxybuttersäure in einer Menge von 0,007 mg/100 g bis 7 g/100 g der Zusammensetzung auf einer Trockengewichtsbasis vorhanden ist; oder
wobei 3-Hydroxybuttersäure in einer Menge von 0,003 mg bis 3 g pro Portion vorhanden ist.

2. Nährstoffzusammensetzung zur Verwendung nach Anspruch 1, wobei es sich bei dem Subjekt um einen Säugling (Kind jünger als 12 Monate) oder ein Kleinkind (zwischen 1 Jahr und unter 3 Jahren) handelt.

3. Nährstoffzusammensetzung zur Verwendung nach Anspruch 1, wobei es sich bei der Nährstoffzusammensetzung um eine Wachstumsmilch handelt und wobei es sich bei dem Subjekt um ein Kind im Alter von 3 Jahren bis unter 8 Jahren handelt.

4. Nährstoffzusammensetzung zur Verwendung nach einem der vorstehenden Ansprüche, wobei es sich bei der Verbesserung der gastrointestinalen Barriere um eine verbesserte Barrierestruktur, eine verbesserte Barrierefunktion, einen verbesserten Barriereschutz und/oder eine verbesserte Barrierereparatur handelt.

5. Nährstoffzusammensetzung zur Verwendung nach einem der vorstehenden Ansprüche, wobei es sich bei der Verbesserung der gastrointestinalen Barriere um die verbesserte Barrierefunktion, die verbesserte Barrierestruktur und/oder den verbesserten Barriereschutz handelt.

6. Nährstoffzusammensetzung zur Verwendung nach einem der vorstehenden Ansprüche, wobei es sich bei der Verbesserung der gastrointestinalen Barriere um den verbesserten Barriereschutz handelt.

7. Nährstoffzusammensetzung zur Verwendung nach Anspruch 1, wobei die Verwendung der Prävention der Barrieredurchlässigkeit dient und wobei es sich bei der Prävention der Barrieredurchlässigkeit um die Prävention eines Migrierens von Krankheitserregern, Allergenen und/oder toxischen Verbindungen aus dem Darm durch die Darmbarriere in den Körper handelt.

8. Nährstoffzusammensetzung zur Verwendung nach Anspruch 1, wobei das Subjekt eine beeinträchtigte Mikrobiota aufweist und wobei es sich bei der beeinträchtigten Mikrobiota um eine unausgeglichene Mikrobiota handelt, die einen ungewöhnlich niedrigen Anteil an *Bifidobacterium,* vorzugsweise einen ungewöhnlich niedrigen Anteil an *Bifidobacterium longum* und/oder Bifidobacterium bifidum, mehr bevorzugt einen ungewöhnlich niedrigen Anteil an *Bifidobacterium bifidum und*/*oder Bifidobacterium longum* Subsp. *infantis,* aufweist.

9. Nährstoffzusammensetzung zur Verwendung nach Anspruch 1 oder 8, wobei die beeinträchtigte Mikrobiota weniger als 80 %, vorzugsweise weniger als 70 %, mehr bevorzugt weniger als 60 % Actinobacteriaceae und noch mehr bevorzugt Bifidobacterium aufweist, wobei die Prozentsätze zahlenmäßig basierend auf den Gesamtbakterien in der Mikrobiota definiert sind.

10. Nährstoffzusammensetzung zur Verwendung nach einem der Ansprüche 8 bis 9, wobei die beeinträchtigte Mikrobiota weniger als 20 %, vorzugsweise weniger als 15 %, mehr bevorzugt weniger als 10 % *Bifidobacterium bifidum* aufweist, wobei die Prozentsätze zahlenmäßig basierend auf den Gesamtbakterien in der Mikrobiota definiert sind.

11. Nährstoffzusammensetzung zur Verwendung nach einem der vorstehenden Ansprüche, wobei es sich bei dem Subjekt um einen Säugling, der aus der Gruppe ausgewählt ist, bestehend aus frühgeborenen Säuglingen, Säuglingen, die klein für ihr Gestationsalter sind, und Säuglingen mit niedrigem Geburtsgewicht, vorzugsweise um einen frühgeborenen Säugling handelt.

12. Nährstoffzusammensetzung zur Verwendung nach einem der vorstehenden Ansprüche, wobei 3-Hydroxybuttersäure in einer Menge von 0,1 mg/l bis 1 g/l der Zusammensetzung oder in einer Menge von 0,07 mg/100 g bis 0,7 g/100 g der Zusammensetzung auf einer Trockengewichtsbasis vorhanden ist; und/oder wobei 3-Hydroxybuttersäure in einer Menge von 0,03 mg bis 0,3 g pro Portion vorhanden ist.

13. Nährstoffzusammensetzung zur Verwendung nach einem der vorstehenden Ansprüche, wobei 3-Hydroxybuttersäure in einer solchen Menge bereitgestellt wird, dass ein normaler Verzehr der Nährstoffzusammensetzung dem Subjekt, das sie zu sich nimmt, eine Gesamttagesdosis von 0,006 mg bis 6 g pro Tag bereitstellen würde.

14. Nährstoffzusammensetzung zur Verwendung nach einem der Ansprüche 1 und 4 bis 13, wobei es sich bei der Nährstoffzusammensetzung um eine Säuglingsanfangsnahrung, eine Starter-Säuglingsanfangsnahrung, eine Folge- oder Nachfolgenahrung, eine Wachstumsmilch, ein Babynahrungsmittel, eine Säuglingsgetreidezusammensetzung, ein Stärkungsmittel wie ein Stärkungsmittel für menschliche Milch, ein Ergänzungsmittel, ein Gesundheitsprodukt, ein medizinisches Nahrungsmittel oder eine Sondenernährungszusammensetzung handelt.

15. Nährstoffzusammensetzung zur Verwendung nach Anspruch 2, wobei es sich bei der Nährstoffzusammensetzung um eine Säuglingsanfangsnahrung, eine Starter-Säuglingsanfangsnahrung, eine Folge- oder Nachfolgenahrung, eine Wachstumsmilch, ein Babynahrungsmittel, eine Säuglingsgetreidezusammensetzung, ein Stärkungsmittel wie ein Stärkungsmittel für menschliche Milch oder ein Ergänzungsmittel handelt.

## Revendications

1. Composition nutritionnelle comprenant de l'acide 3-hydroxybutyrique pour utilisation dans l'amélioration de la barrière gastro-intestinale chez un sujet ;
où ladite utilisation est choisie dans le groupe constitué de l'amélioration de la prévention du dysfonctionnement de la barrière, la prévention du manque d'étanchéité de la barrière, la protection de la structure des jonctions serrées et la protection de l'intégrité de la paroi épithéliale intestinale ; et/ou
où le sujet a un microbiote altéré ;
où l'acide 3-hydroxybutyrique est présent en une quantité de 0,01 mg/L à 10 g/L de la composition, ou
où l'acide 3-hydroxybutyrique est présent en une quantité de 0,007 mg/100 g à 7 g/100 g de la composition sur une base de poids sec ; ou
où l'acide 3-hydroxybutyrique est présent en une quantité de 0,003 mg à 3 g par portion.

2. Composition nutritionnelle pour utilisation selon la revendication 1, où le sujet est un nourrisson (enfant de moins de 12 mois) ou un jeune enfant (entre 1 an et moins de 3 ans).

3. Composition nutritionnelle pour utilisation selon la revendication 1, où la composition nutritionnelle est un lait de croissance et où le sujet est un enfant âgé de 3 ans à moins de 8 ans.

4. Composition nutritionnelle pour utilisation selon l'une quelconque des revendications précédentes, où ladite amélioration de la barrière gastro-intestinale est une amélioration de la structure de la barrière, une amélioration de la fonction de la barrière, une amélioration de la protection de la barrière et/ou une amélioration de la réparation de la barrière.

5. Composition nutritionnelle pour utilisation selon l'une quelconque des revendications précédentes, où ladite amélioration de la barrière gastro-intestinale est une amélioration de la fonction de la barrière, une amélioration de la structure de la barrière, et/ou une amélioration de la protection de la barrière.

6. Composition nutritionnelle pour utilisation selon l'une quelconque des revendications précédentes, où ladite amélioration de la barrière gastro-intestinale est une amélioration de la protection de la barrière.

7. Composition nutritionnelle pour utilisation selon la revendication 1, où ladite utilisation est destinée à la prévention du manque d'étanchéité de la barrière et où ladite prévention du manque d'étanchéité de la barrière consiste à empêcher des agents pathogènes, des allergènes et/ou des composés toxiques de migrer de l'intestin vers l'organisme à travers la barrière intestinale.

8. Composition nutritionnelle pour utilisation selon la revendication 1, où le sujet a un microbiote altéré et où le microbiote altéré est un microbiote déséquilibré ayant une proportion anormalement faible de *Bifidobacterium,* de préférence une proportion anormalement faible de *Bifidobacterium longum* et/ou de *Bifidobacterium bifidum,* plus préférablement une proportion anormalement faible de *Bifidobacterium bifidum* et/ou de *Bifidobacterium longum* subsp. *infantis.*

9. Composition nutritionnelle pour utilisation selon la revendication 1 ou 8, où le microbiote altéré a moins de 80 %, de préférence moins de 70 %, plus préférablement moins de 60 *%* d'*Actinobacteriaceae* et encore plus préférablement de *Bifidobacterium,* les pourcentages étant définis en nombre par rapport au nombre total de bactéries dans le microbiote.

10. Composition nutritionnelle pour utilisation selon l'une quelconque des revendications 8 à 9, où le microbiote altéré a moins de 20 %, de préférence moins de 15 %, plus préférablement moins de 10 % de *Bifidobacterium bifidum,* les pourcentages étant définis en nombre par rapport au nombre total de bactéries dans le microbiote.

11. Composition nutritionnelle pour utilisation selon l'une quelconque des revendications précédentes, où le sujet est un nourrisson choisi dans le groupe constitué de nourrissons prématurés, nourrissons de petite taille pour l'âge gestationnel et nourrissons de faible poids à la naissance, de préférence un nourrisson prématuré.

12. Composition nutritionnelle pour utilisation selon l'une quelconque des revendications précédentes, où l'acide 3-hydroxybutyrique est présent en une quantité de 0,1 mg/L à 1 g/L de la composition ou en une quantité de 0,07 mg/100 g à 0,7 g/100 g de la composition sur une base de poids sec ; et/ou où l'acide 3-hydroxybutyrique est présent en une quantité de 0,03 mg à 0,3 g par portion.

13. Composition nutritionnelle pour utilisation selon l'une quelconque des revendications précédentes, où l'acide 3-hydroxybutyrique est fourni en une quantité telle qu'une consommation normale de la composition nutritionnelle apporterait au sujet qui la consomme une dose quotidienne totale de 0,006 mg à 6 g par jour.

14. Composition nutritionnelle pour utilisation selon l'une quelconque des revendications 1 et 4 à 13, où ladite composition nutritionnelle est une préparation pour nourrissons, une préparation pour nourrissons de premier âge, une préparation de suite ou de transition, un lait de croissance, un aliment pour bébés, une composition de céréales pour nourrissons, un fortifiant tel qu'un fortifiant de lait maternel, un complément, un produit pour soins de santé, un aliment médical ou une composition pour alimentation par sonde.

15. Composition nutritionnelle pour utilisation selon la revendication 2, où ladite composition nutritionnelle est une préparation pour nourrissons, une préparation pour nourrissons de premier âge, une préparation de suite ou de transition, un aliment pour bébés, une composition de céréales pour nourrissons, un fortifiant tel qu'un fortifiant de lait maternel ou un complément.
